Europäisches Patentamt

European Patent Office

Office européen des brevets

(19)

(11) Publication number: **0 274 445 B1**

# EUROPEAN PATENT SPECIFICATION

(12)

(45) Date of publication of patent specification: **28.07.93**  (51) Int. Cl.5: **C12N 5/00**

(21) Application number: **88300121.6**

(22) Date of filing: **08.01.88**

(54) **A serum-free growth medium additive and a use thereof.**

(30) Priority: **09.01.87 NO 870095**

(43) Date of publication of application:
**13.07.88 Bulletin  88/28**

(45) Publication of the grant of the patent:
**28.07.93 Bulletin  93/30**

(84) Designated Contracting States:
**AT BE CH DE FR GB LI NL SE**

(56) References cited:

CHEMICAL ABSTRACTS, vol. 101, no. 7, 13th
August 1984, page 394, abstract no. 52317g,
Columbus, Ohio, US; A. HRADILEK et al.:
"Iron uptake into HeLa cells adapted for
growth in serum free medium", & STRUCT.
FUNCT. IRON STORAGE TRANSP. PROTEINS,
PROC. INT. CONF., 6th 1983, 121-2

BIOLOGICAL ABSTRACTS, vol. 69, no. 4,
1980, abstract no. 24569, Biological Ab-
stracts, Inc., Philadelphia, PA, US; M.
MOSKOWITZ et al.: "Growth and G1 arrest of
sarcoma virus transformed cells in serum
free media.", & J CELL PHYSIOL 100(3):
589-602. 1979

(73) Proprietor: **Medi-Cult A/S**
**Lersoe Park Alle 42**
**DK-2100 Copenhagen(DK)**

(72) Inventor: **Bertheussen, Kjell**
**Eidhaugsvingen 8**
**N-9100 Kvaloysletta(NO)**

(74) Representative: **Holmes, Michael John**
**Frank B. Dehn & Co. European Patent Attor-**
**neys Imperial House 15-19 Kingsway**
**London, WC2B 6UZ, (GB)**

EP 0 274 445 B1

## Description

The present invention concerns a serum-free medium additive which may be used when culturing cells which require iron in the growth medium. The serum-free growth medium additive according to the invention may additionally be used to test the quality of growth media.

In industry and research there has for a long time been a great need for serum-free media and growth cultures since the supply of serum to such media in addition to the compounds which are necessary for growth also adds to the solutions compounds that negatively influence the majority of experiments where accuracy of the results are of great importance. This is valid within diciplines such as cellular immunology, biotechnology, in vitro fertilization, organ transplants, cancer research and storing and transfusion of blood.

The media of physiological solutions which are used today in research, industry and clinical work are based on recipes up to 100 years old and thus represent a stand-still in this field of biological research. Media used hitherto for cell cultures consists most frequently of a so-called commercial basal medium to which there is added approximately 10% heat-inactivated serum. This very old method involves the following disadvantages:

1) The heat treatment needed to prevent the lytic effect of the serum on cells, has a denaturing effect on important serum components.

2) Different serum batches have different properties on account of their origin, which adds undesired variations to the behaviour of the cell culture in vitro.

3) Serum contains several unknown substances with unknown and uncontrolled effects on cells.

4) Serum is an unphysiological fluid for the majority of cells since the cells are adapted to the so-called tissue-fluid in the body and this fluid has a different content of various compounds compared to serum.

5) Antibodies in serum may bind cells and interfere with experiments.

6) Serum prevents the study of the synthesis by cells of serum factors in vitro since the background level in serum of such factors is relatively high.

By using the serum-free additive according to the present invention, the above disadvantages are avoided, and such a medium will in addition be very well suited for culturing and storing cells and tissue in vitro. In addition, a complete medium with this additive will be precisely defined, be serum free and protein-free (except for a possible small quantity of insulin which is required by some cells, and which is used where necessary) and contain iron and trace elements in the form of stabilized chelated elements or compounds with transferrin or lipids being present. It is also very inexpensive to produce the additive according to the invention.

The main problem of previous serum-free media (see N.N. Iscove and F. Melchers, "Complete Replacement of Serum by Albumin, Transferrin, and Soybean Lipid in Cultures of Lipopolysaccharide-reactive B-lymphocytes", J.Exp.Med., Vol. 147, pp. 923-933 (1978); R.G. Ham, "Importance of the Basal Medium in the Design of Hormonally Defined Media", in: Growth of Cells in Hormonally Defined Media, Cold Spring Harbor Conference on Cell Proliferation, Vol. 9, pp 39-60 (1982); D. Barnes and G. Sato, "Methods for Growth of Cultured Cells in Serum-free Medium", Anal. Biochem. 102, pp. 255-270 (1980)) is that they are not suitable for application to a randomly chosen cell culture on account of two circumstances:

1) The presence of physiological amounts of bicarbonate and $Ca^{2+}/Mg^{2+}$ ions will precipitate iron and trivalent trace metals from almost any known chelant (i.e. chelating agent) system at 37°C and pH 7-8 (except transferrin). This conclusion arises from the inventor's personal work (unpublished).

2) The concentration of trace metals including toxic compounds in a medium is not controlled, and will vary significantly depending on the composition and the individual properties of the water and impurities therein. The only solution to this problem is to provide a completely stable metal ion buffer comprising a balanced solution of all the necessary trace elements and metals, and thus the toxic metals will be absorbed by the buffer (the toxic effect will become balanced away).

Since transferrin is expensive and species-specific, the medium according to the invention was produced, on the bases of a Fe/trace element buffer of synthetic non-protein chelants and such that the main problem, precipitation of metals at 37°C (see above), was solved by adding certain chelants which in combination in solution have very different and unique properties compared to each chelant alone.

The second problem, viz. the presentation of iron to the cell membrane and the cell, was solved by the addition of aurin tricarboxylic acid to the additive. Even if Ethylenediaminetetraacetic acid (EDTA)/citrate renders the metal solution completely stable, various animal or human cells will not grow because of iron deficiency. It has been found that the combination EDTA/citrate/aurintricarboxylic acid produces outstanding chelating and iron presenting properties which previously have not been described. (In this connection it is of no consequence that EDTA alone previously has been used as an iron chelant for the growth of plant cells at pH 5.0 under conditions where metal precipitation does not represent any problem; see: P. Kruse

2

and M.K. Petterson, "Tissue Culture Methods and Applications", Acad. Press (1973); J.R. Stein, "Handbook of Physiological Methods", Cambridge University Press (1973).

In accordance with this, the serum-free growth medium additive of the invention comprises

a) an iron chelate,

b) aurintricarboxylic acid,

c) optionally an alkali metal-EDTA, and

d) optionally additional trace elements,

the medium preferably buffering in the pH range of 7.0 to 7.8.

In connection with cell growth it has been found that a ratio EDTA to citric acid (citrate) of 1:3 as an example was very advantageous. It should also be mentioned briefly that cell growth in media usually is conducted at pH 7.4 but that pH in the final medium according to the present invention may also differ from this, depending on the growth requirements of the various cells.

An additive according to the present invention may, e.g., be prepared by adding a concentrated solution of each of the components to pure water (it is of great importance to use water of highest possible purity such as for instance "Travenol" sterile water, when preparing the medium according to the invention). An example of such a concentrated mixture is given below, where there is produced a composition which may be used in a 1000-fold concentration:

|  |  |  | Weight per 100 ml of water |
|---|---|---|---|
| 3 mM | Fe-EDTA[1] | | 120.1 mg |
| 3 mM | Aurintricarboxylic acid[2] | | 126.7 mg |
| 1 mM | Na$_2$-EDTA | | 37.2 mg |
| 15 mM | Citric acid | | 315.0 mg |
| 25 mM | Na$_3$-citrate | | 735.0 mg |
| (optionally 1% trace elements [3]) | | | |

[1] Ethylenediaminetetraacetic acid Fe(III)-Na chelate dihydrate (Koch-Light No. 2529-00)

[2] Sigma A 1895

[3] May be as the trace element composition shown below.

The pH of the composition is preferably adjusted to 4.5-5.0.

An optional addition of trace elements may, e.g., be carried out be the aid of a stock solution which may be prepared as follows. That solution will have a 100 000 fold concentration of that to be used in the medium. When the solution defined below is prepared, Zn, Cu and chelants (EDTA and citrate) should be added prior to adjusting pH to 5.0 with 5-10N NaOH, after which the other components are added. The final pH should be between 4.0 and 4.5.

| Trace metal (conc.) | | | Salt | Weight per 100 ml water | |
|---|---|---|---|---|---|
| 10 | mM | Zn | $ZnSO_4.7H_2O$ | 287.5 | mg |
| 2 | mM | Cu | $CuSO_4.5H_2O$ | 49.9 | mg |
| 0.1 | mM | Mn | $MnSO_4.H_2O$ | 1.7 | mg |
| 0.02 | mM | Ni | $Ni(NO_3)_2.6H_2O$ | 0.58 | mg |
| 0.2 | mM | Al | $NH_4Al(SO_4)_2.12H_2O$ | 9.2 | mg |
| 0.1 | mM | Cr | $KCr(SO_4)_2.12H_2O$ | 5.0 | mg |
| 0.02 | mM | Co | $CoCl_2.6H_2O$ | 0.48 | mg |
| 1 | mM | Se | $SeO_2$ | 11.1 | mg |
| 14 | mM | $Na_2$-EDTA | | 521.1 | mg |
| 1 | mM | $Na_3$-citrate | | 29.4 | mg |

It is of course to be understood that such a composition of trace elements may contain elements other than those given in the table above, depending on the requirements of the tissue of cell types to be used.

The additive according to the invention may, besides the components mentioned hereinbefore contain growth stimulating and necessary compounds for cell growth. Such compounds may comprise for instance the surfactant "Pluronic" F 68 (20 mg/l), D-glucose (2.5 g/l), L-glutamine (2 mM), Na-pyruvate (1 mM), insulin (0.5 mg/l) or ethanolamine (20 $\mu$M). All the concentrations shown in brackets refer to final concentrations. A composition for the addition of the compounds mentioned (except of ethanolamine which is a liquid at room temperature) may be produced as a dry product and as such this may be stored during long periods of time, up to several years. Ethanolamine may, if necessary, be added to the medium to a desired concentration when using the composition. Ethanolamine is most frequently used in experiments with hybridoma cells when producing monoclonal antibodies.

A preferred basal medium to which the growth medium additive according to the present invention may be added, is RPMI 1640 containing 2.5g/l $NaHCO_3$ and 20mM HEPES (N-2-hydroxyethylpiperazine-N,N'-2-ethanesulphonic acid).

Preferred cell cultures for which the serum-free additive according to the invention have proven to be especially useful, is the L 929 mouse fibroblast cell line, the HeLa human tumor cell line, various mouse and human hybridoma cells which all produce monoclonal anti-bodies in quantities corresponding to growth in serum-containing cultures, and human monocytes which differentiate to developed macrophages during 7 days while keeping their complement receptors.

A particularly preferred use of the present additive is carried out with fast-growing types of cells for testing the quality of various types of media. By mixing the serum-free medium additive according to the invention with RPMI 1640 and adding the medium to be tested in the ratio serum-free medium additive + RPMI 1640:unknown medium = 1:1, and then adding the fast-growing cells in a number of about 30 000 cells/ml, there will, when no toxic compounds are present in the medium to be tested, be observed an increase in the number of cells after 4 days of approximately 10 times more cells than the original number. If, however, toxic compounds are present in the unknown medium, cell growth will not be observed. This thus is a very simple quality test which is extremely sensitive because it is performed without serum. False results are avoided because the fact that serum binds and masks toxic impurities is avoided.

The growth medium additive according to the invention together with fast-growing cells as mentioned may be incorporated into a test-kit comprising, e.g., the additive (serum substitute) according to the invention, frozen cells (for instance on frozen carbondioxide) and optionally a medium (e.g. RPMI 1640) and culture equipment of plastics or other suitable material.

Preferred fast-growing cells for use in testing full growth media are cells of a fast-growing cell line of which a cell culture has been deposited at the European Collection of Animal Cell Cultures, Porton Down, Salisbury, Wiltshire SP4 OJG on November 20, 1986, under the accession number 86112001. This cell type is a special kind of fast-growing cell type (of all-mouse origin) of hybridoma cells prepared by the fusion of

EP 0 274 445 B1

$p_3U_1$ tumor cells with B-lymphocytes. This cell line has proven to be especially useful at quality testing of full growth media and as such is a particular aspect of the present invention.

**Claims**

1. A serum-free growth medium additive, characterised in that it comprises an iron chelate and aurin tricarboxylic acid.

2. A serum-free growth medium additive as claimed in claim 1, wherein said iron chelate comprises a composition of Fe-EDTA and citric acid.

3. A serum-free growth medium additive as claimed in claim 2, wherein said iron chelate comprises a composition of Fe-EDTA and citric acid in a ratio of 1:3.

4. A serum-free growth medium additive, as claimed in any one of claims 1 to 3, wherein said additive additionally comprises an alkali metal-EDTA complex.

5. A serum-free growth medium additive, as claimed in any one of claims 1 to 4, wherein said additive additionally comprises trace elements.

6. A serum-free growth medium additive, as claimed in claim 5 wherein said trace elements comprise the metals Zn, Cu, Mn, Ni, Al, Cr, Co and Se.

7. A serum-free growth medium additive, as claimed in any one of claims 1 to 6 wherein the medium is buffered in the pH range of 7.0 to 7.8.

8. A serum-free growth medium additive as claimed in any one of claims 1 to 7, wherein the individual components are present in amounts physiologically acceptable for cell growth.

9. A serum-free growth medium additive as claimed in any one of claims 1 to 8, wherein said additive additionally comprises serum free growth factors.

10. A serum-free growth medium additive as claimed in claim 9, wherein said serum-free growth factors are selected from "Pluronic F68, D-glucose, L-glutamine, Na-pyruvate, insulin and ethanolamine.

11. A serum-free growth medium additive as claimed in any one of claims 1 to 10, wherein said additive comprises a basal medium for cell cultures.

12. A serum-free growth medium additive as claimed in claim 11, wherein said basal medium is RPMI 1640/HEPES/NaHCO$_3$ in a concentration of 2.5g/l NaHCO$_3$ and 20mM HEPES.

13. A serum-free growth medium additive as claimed in any one of claims 1 to 10, wherein said additive has the form of a concentrate.

14. A serum-free growth medium additive as claimed in claim 13, wherein said concentrate is buffered in the ph range of 4.5 to 5.0.

15. A serum-free growth medium additive as claimed in any one of claims 1 to 10, wherein said additive has the form of a dry-matter composition.

16. A serum-free growth medium additive as claimed in claim 13 wherein said additive comprises
2.4-3.6 mM Fe-EDTA,
2.4-3.6 mM aurintricarboxylic acid,
0.8-1.2 mM Na$_2$-EDTA,
12-18 mM citric acid,
20-30 mM Na$_3$-citrate

5

**17.** A serum-free growth medium additive as claimed in claim 14, wherein said additive additionally comprises the ion concentrations

0.08-0.12 mM $Zn^{2+}$

0.016-0.024 mM $Cu^{2+}$

0.0008-0.0012 mM $Mn^{2+}$

0.00016-0.00024 mM $Ni^{2+}$

0.0016-0.0024 mM $Al^{3+}$

0.0008-0.0012 mM $Cr^{3+}$

0.00016-0.00024 mM $Co^{2+}$ and

0.008-0.012 mM $Se^{4+}$.

**18.** A growth medium containing a serum-free growth medium additive as claimed in any of claims 1 to 17 together with a hybridoma cell-line formed between $P_3U_1$ tumor cells and B-lymphocytes (deposit number ECACC 86112001).

**19.** Use of the serum-free growth medium additive claimed in any one of claims 1 to 15 for testing the quality of full growth media.

**20.** Use of the serum-free growth additive as claimed in claim 19, in combination with hybridoma cells as defined in claim 18.

**21.** A test kit for use in evaluating the quality of full growth media, wherein said test kit comprises a serum-free growth medium additive as claimed in any one of claims 1 to 15, and frozen hybridoma cells as defined in claim 18.

**Patentansprüche**

**1.** Serumfreies Additiv für ein Wachstumsmedium, dadurch gekennzeichnet, daß es ein Eisen-Chelat und Aurintricarbonsäure umfaßt.

**2.** Serumfreies Additiv für ein Wachstumsmedium nach Anspruch 1, Worin das Eisenchelat eine Zubereitung aus Fe-EDTA und Citronensäure umfaßt.

**3.** Serumfreies Additiv für ein Wachstumsmedium nach Anspruch 2, worin das Eisenchelat eine Zubereitung aus Fe-EDTA und Citronensäure in einem Verhältnis 1 : 3 umfaßt.

**4.** Serumfreies Additiv für ein Wachstumsmedium nach einem der Ansprüche 1 bis 3, worin das Additiv außerdem einen Alkalimetall-EDTA-Komplex umfaßt.

**5.** Serumfreies Additiv für ein Wachstumsmedium nach einem der Ansprüche 1 bis 4, worin das Additiv außerdem Spurenelemente umfaßt.

**6.** Serumfreies Additiv für ein Wachstumsmedium nach Anspruch 5, worin die Spurenelemente die Metalle Zn, Cu, Mn, Ni, Al, Cr, Co und Se umfassen.

**7.** Serumfreies Additiv für ein Wachstumsmedium nach einem der Ansprüche 1 bis 6, worin das Medium in einem pH-Wert-Bereich von 7,0 bis 7,8 gepuffert ist.

**8.** Serumfreies Additiv für ein Wachstumsmedium nach einem der Ansprüche 1 bis 7, worin die einzelnen Komponenten in Mengen zugegen sind, die physiologisch für ein Zellwachstum annehmbar sind.

**9.** Serumfreies Additiv für ein Wachstumsmedium nach einem der Ansprüche 1 bis 8, worin das Additiv außerdem serumfreie Wachstumsfaktoren umfaßt.

**10.** Serumfreies Additiv für ein Wachstumsmedium nach Anspruch 9, worin die serumfreien Wachstumsfaktoren gewählt sind aus "Pluronic" F68, D-Glucose, L-Glutamin, Natriumpyruvat, Insulin und Ethanolamin.

**11.** Serumfreies Additiv für ein Wachstumsmedium nach einem der Ansprüche 1 bis 10, worin das Additiv ein Grundmedium für Zellkulturen umfaßt.

**12.** Serumfreies Additiv für ein Wachstumsmedium nach Anspruch 11, worin das Grundmedium RPMI 1640/HEPES/NaHCO$_3$ in einer Konzentration von 2,5 g NaHCO$_3$/1 und 20 mM HEPES ist.

**13.** Serumfreies Additiv für ein Wachstumsmedium nach einem der Ansprüche 1 bis 10, worin das Additiv in Form eines Konzentrats vorliegt.

**14.** Serumfreies Additiv für ein Wachstumsmedium nach Anspruch 13, worin das Konzentrat in einem pH-Wert-Bereich von 4,5 bis 5,0 gepuffert ist.

**15.** Serumfreies Additiv für ein Wachstumsmedium nach einem der Ansprüche 1 bis 10, worin das Additiv in Form einer Trockenmasse-Zubereitung vorliegt.

**16.** Serumfreies Additiv für ein Wachstumsmedium nach Anspruch 13, worin das Additiv umfaßt:
2,4 - 3,6 mM Fe-EDTA,
2,4 - 3,6 mM Aurintricarbonsäure,
0,8 - 1,2 mM Na$_2$-EDTA,
12 - 18 mM Citronensäure und
20 - 30 mM Na$_3$-citrat.

**17.** Serumfreies Additiv für ein Wachstumsmedium nach Anspruch 14, worin das Additiv außerdem die folgenden Ionen in den angegebenen Konzentrationen umfaßt:
0,08 -0,12 mM Zn$^{2+}$,
0,016 - 0,024 mM Cu$^{2+}$,
0,0008 - 0,0012 mM Mn$^{2+}$,
0,00016 - 0,00024 mM Ni$^{2+}$,
0,0016 - 0,0024 mM Al$^{3+}$,
0,0008 - 0,0012 mM Cr$^{3+}$,
0,00016 - 0,00024 mM Co$^{2+}$, und
0,008 - 0,012 mM Se$^{4+}$.

**18.** Wachstumsmedium, das ein serumfreies Additiv für ein Wachstumsmedium nach einem der Ansprüche 1 bis 17 zusammen mit einer Hybridoma-Zellinie enthält, die zwischen P$_3$U$_1$-Tumorzellen und B-Lymphocyten (Hinterlegungsnummer ECACC 86112001) gebildet ist.

**19.** Verwendung des serumfreien Additivs für ein Wachstumsmedium nach einem der Ansprüche 1 bis 15 für Tests der Qualität vollständiger Wachstumsmedien.

**20.** Verwendung des serumfreien Additivs für ein Wachstumsmedium nach Anspruch 19 in Kombination mit Hybridoma-Zellen, Wie sie in Anspruch 18 definiert sind.

**21.** Testkit zur Verwendung bei der Bewertung der Qualität vollständiger Wachstumsmedien, worin das Testkit ein serumfreies Additiv für ein Wachstumsmedium nach einem der Ansprüche 1 bis 15 und gefrorene Hybridoma-Zellen umfaßt, wie sie in Anspruch 18 definiert sind.

**Revendications**

**1.** Un additif de milieu de culture exempt de sérum, caractérisé en ce qu'il comprend un chélate de fer et de l'acide aurintricarboxylique.

**2.** Un additif de milieu de culture exempt de sérum selon la revendication 1, dans lequel ledit chélate de fer comprend une composition de Fe-EDTA et d'acide citrique.

**3.** Un additif de milieu de culture exempt de sérum selon la revendication 2, dans lequel ledit chélate de fer comprend une composition de Fe-EDTA et d'acide citrique dans un rapport de 1:3.

**4.** Un additif de milieu de culture exempt de sérum selon l'une quelconque des revendications 1 à 3, dans lequel ledit additif comprend de plus un complexe EDTA-métal alcalin.

**5.** Un additif de milieu de culture exempt de sérum selon l'une quelconque des revendications 1 à 4, dans lequel ledit additif comprend de plus des éléments à l'état de traces.

**6.** Un additif de milieu de culture exempt de sérum selon la revendication 5, dans lequel lesdits éléments à l'état de traces comprennent les métaux Zn, Cu, Mn, Ni, Al, Cr, Co et Se.

**7.** Un additif de milieu de culture exempt de sérum selon l'une quelconque des revendications 1 à 6, dans lequel le milieu est tamponné dans la gamme de pH de 7,0 à 7,8.

**8.** Un additif de milieu de culture exempt de sérum selon l'une quelconque des revendications 1 à 7, dans lequel les composants individuels sont présents dans des quantités physiologiquement acceptables pour la culture de cellules.

**9.** Un additif de milieu de culture exempt de sérum selon l'une quelconque des revendications 1 à 8, dans lequel ledit additif comprend de plus des facteurs de croissance exempts de sérum.

**10.** Un additif de milieu de culture exempt de sérum selon la revendication 9, dans lequel lesdits facteurs de culture exempts de sérum sont choisis parmi le "Pluronic" F68, le D-glucose, la L-glutamine, le pyruvate de sodium, l'insuline et l'éthanolamine.

**11.** Un additif de milieu de culture exempt de sérum selon l'une quelconque des revendications 1 à 10, dans lequel ledit additif comprend un milieu de base pour des cultures de cellules.

**12.** Un additif de milieu de culture exempt de sérum selon la revendication 11, dans lequel ledit milieu de base est RPMI 1640/HEPES/NaHCO$_3$ dans une concentration de 2,5 g/l de NaHCO$_3$ et de 20 mM de HEPES.

**13.** Un additif de milieu de culture exempt de sérum selon l'une quelconque des revendications 1 à 10, dans lequel ledit additif se présente sous la forme d'un concentré.

**14.** Un additif de milieu de culture exempt de sérum selon la revendication 13, dans lequel ledit concentré est tamponné dans la gamme de pH de 4,5 à 5,0.

**15.** Un additif de milieu de culture exempt de sérum selon l'une quelconque des revendications 1 à 10, dans lequel ledit additif se présente sous la forme d'une composition de matière sèche.

**16.** Un additif de milieu de culture exempt de sérum selon la revendication 13, dans lequel ledit additif comprend
Fe-EDTA 2,4-3,6 mM,
acide aurintricarboxylique 2,4-3,6 mM,
Na$_2$-EDTA 0,8-1,2 mM,
acide citrique 12-18 mM,
Na$_3$-citrate 20-30 mM.

**17.** Un additif de milieu de culture exempt de sérum selon la revendication 14, dans lequel ledit additif comprend de plus les concentrations ioniques
0,08-0,12 mM de Zn$^{2+}$
0,016-0,024 mM de Cu$^{2+}$
0,0008-0,0012 mM de Mn$^{2+}$
0,00016-0,00024 mM de Ni$^{2+}$
0,0016-0,0024 mM de Al$^{3+}$
0,0008-0,0012 mM de Cr$^{3+}$
0,00016-0,00024 mM de Co$^{2+}$ et
0,008-0,012 mM de Se$^{4+}$.

**18.** Un milieu de culture contenant un additif de milieu de culture exempt de sérum selon l'une quelconque des revendications 1 à 17, conjointement avec une lignée de cellules hybridomes formée entre des cellules tumorales $P_3U_1$ et des B-lymphocytes (numéro de dépôt ECACC 86112001).

**19.** Utilisation de l'additif de milieu de culture exempt de sérum selon l'une quelconque des revendications 1 à 15 pour contrôler la qualité de milieux de cultures globaux.

**20.** Utilisation de l'additif de milieu de culture exempt de sérum selon la revendication 19, en combinaison avec des cellules hybridomes telles que définies à la revendication 18.

**21.** Un ensemble de contrôle utilisable pour évaluer la qualité de milieux de culture globaux, dans lequel ledit ensemble de contrôle comprend un additif de milieu de culture exempt de sérum selon l'une quelconque des revendications 1 à 15 et des cellules hybridomes congelées telles que définies à la revendication 18.